# EUROPEAN PATENT APPLICATION

(11) **EP 4 282 342 A1**
(43) Date of publication of application: **29.11.2023**
(21) Application number: 22742789.5
(22) Date of filing: 18.01.2022
(51) Int. Cl.: A61B 10/00

(54) **SPECIMEN COLLECTION KIT**

(30) Priority: 20.01.2021 KR 20210007766
(71) Applicant: Bionlifescience, Inc., Namyangju-si, Gyeonggi-do 12106 (KR)
(72) Inventor: GOH, Chang Wook, Namyangju-si Gyeonggi-do 12095 (KR); JEONG, Joong Hwan, Bucheon-si Gyeonggi-do 14500 (KR); KIM, Bong Yoon, Goyang-si Gyeonggi-do 10469 (KR)
(74) Representative: dompatent von Kreisler Selting Werner - Partnerschaft von Patent- und Rechtsanwälten mbB
(86) International application number: PCT/KR2022/000872
(87) International publication number: WO 2022/158813

(57) **Abstract**

The present invention may provide a specimen collection kit characterized by comprising: a tube body; a coupling member detachably coupled to one side of the tube body; a cover member detachably coupled to the other side of the tube body; and a specimen guide part which is provided on the inner surface of the tube body, prevents an outlet from being clogged by a collection means, and guides the flow of the specimen to the outlet. According to the above configuration, blocking of the outlet by a swab is prevented, and thus the specimen can be collected through the outlet without difficulty. Since a maximal amount of the specimen can be acquired when performing a rapid diagnosis, the effects of extraction and diagnostic testing can be enhanced. Also, since the specimen can be collected by applying only a small amount of pressure, the present invention can reduce fatigue in an inspector and thereby make work easier.

## Description

### [Technical Field]

The present invention relates to a specimen collection kit, and more particularly to a specimen collection kit capable of ensuring smooth extraction of a specimen without blockage attributable to a swab and capable of reducing hand fatigue caused by pressing, thereby facilitating use and extraction.

### [Background Art]

In general, medical examination or inspection is performed in a manner of collecting a specimen, such as blood, saliva, or body organ tissues, from an examinee using a specimen collection kit and carrying out various examinations, such as biochemistry examination, immune serum examination, and hematology examination, on the collected specimen, whereby the health condition and disease of the examinee are diagnosed.

Meanwhile, the aforementioned specimen collection kit may include a swab, which is configured to collect a specimen from an examinee and is generally composed of a head portion and a stick portion, and a body, which is configured to accommodate and store the swab containing a collected specimen therein, and the specimen may be extracted through an outlet formed in the body when an examiner applies external force to the body.

However, such a conventional specimen collection kit has a problem in that, when it is intended to extract the specimen in the state in which the swab is inserted into the body, the specimen is not smoothly extracted due to blockage of the outlet by the head portion of the swab. In particular, in the case of rapid diagnosis in which as large an amount of specimen as possible should be extracted, such blockage adversely affects specimen extraction.

In addition, because the body of the conventional specimen collection means is formed of a hard material, there is a problem in that, when an examiner presses the body to squeeze the swab, the examiner suffers from fatigue due to pressing and thus has difficulty in use and extraction.

### [Disclosure]

### [Technical Problem]

It is an object of the present invention to provide a specimen collection kit capable of ensuring smooth specimen extraction through an outlet while preventing blockage attributable to a swab, thereby enabling extraction of as large an amount of specimen as possible during rapid diagnosis, and capable of enabling an examiner to press and squeeze a head portion with a reduced pressing force, thereby reducing fatigue of the examiner caused by pressing a large number of collection units and further facilitating use and extraction.

### [Technical Solution]

The present invention may provide a specimen collection kit including a tube body including an accommodation space defined therein to accommodate a collection unit containing a collected specimen, an outlet formed in one end thereof so as to communicate with the accommodation space to discharge the specimen, and an insertion hole formed in the other end thereof to allow the collection unit to be inserted into the accommodation space, a cover member detachably coupled to the other end of the tube body to open and close the insertion hole, a coupling member detachably coupled to one end of the tube body to open and close the outlet, and a specimen guiding portion provided on the inner side surface of the tube body so as to be adjacent to the outlet to prevent blockage of the outlet due to the collection unit and to induce flow of the specimen to the outlet.

### [Advantageous Effects]

The specimen collection kit according to the present invention may ensure smooth specimen extraction through an outlet by preventing blockage of the outlet attributable to a swab through a specimen guiding portion, and may enable extraction of as large an amount of specimen as possible during rapid diagnosis, thereby improving extraction and diagnostic check effects.

In addition, the specimen collection kit according to the present invention may be formed of an elastically deformable soft material in order to facilitate a pressing task, whereby an examiner may press and squeeze a collection unit with a reduced pressing force and thus may easily extract a specimen, fatigue of the examiner caused by pressing a large number of collection units may be reduced, and consequently, workability may be improved.

### [Description of Drawings]

FIG. 1 is a perspective view showing the external appearance of a specimen collection kit according to an embodiment of the present invention.
FIG. 2 is an exploded perspective view of the specimen collection kit shown in FIG. 1.
FIG. 3 is a front view showing a tube body in the specimen collection kit shown in FIG. 2.
FIG. 4 is a front view showing another embodiment of the tube body in the specimen collection kit shown in FIG. 3.
FIG. 5 is a front-sectional view showing the internal structure of the tube body, with a collection unit inserted thereinto, in the specimen collection kit shown in FIG. 3.
FIG. 6 is an enlarged cross-sectional view showing the configuration of a specimen guiding portion in the specimen collection kit shown in FIG. 5.
FIG. 7 is a cross-sectional view taken along line VII-VII in the specimen collection kit shown in FIG. 6.
FIG. 8 is a cross-sectional view showing another embodiment of the specimen collection kit shown in FIG. 5, which is provided with a fitting portion.

### [Best Mode]

The present invention will now be described more fully hereinafter with reference to the accompanying drawings, in which various embodiments are shown.

The examples, however, may be embodied in many different forms and should not be construed as being limited to the embodiments set forth herein. It is to be understood that the present invention covers all modifications, equivalents, and alternatives falling within the scope and spirit of the present invention.

It will be understood that, although the terms first, second, etc. may be used herein to describe various elements, these elements should not be limited by these terms. These terms are only used to distinguish one element from another. For example, a first element could be termed a second element, and, similarly, a second element could be termed a first element, without departing from the scope of exemplary embodiments of the present invention. As used herein, the term "and/or" includes any and all combinations of one or more of the associated listed items.

It will be understood that when an element is referred to as being "connected" or "coupled" to another element, it can be directly connected or coupled to the other element, or intervening elements may be present. In contrast, when an element is referred to as being "directly connected" or "directly coupled" to another element, there are no intervening elements present.

The terminology used herein is for the purpose of describing particular embodiments only and is not intended to be limiting of exemplary embodiments of the invention. As used herein, the singular forms are intended to include the plural forms as well, unless the context clearly indicates otherwise. It will be further understood that the term "include" or "have", when used herein, specifies the presence of stated features, integers, steps, operations, elements, components, or combinations thereof, but do not preclude the presence or addition of one or more other features, integers, steps, operations, elements, components, or combinations thereof.

Unless otherwise defined, all terms used herein, which include technical or scientific terms, have the same meanings as those generally appreciated by those skilled in the art. The terms, such as ones defined in common dictionaries, should be interpreted as having the same meanings as terms in the context of pertinent technology, and should not be interpreted as having ideal or excessively formal meanings unless clearly defined in the specification. In addition, in describing the present invention, in order to aid in general understanding, the same reference numerals are used to denote the same elements throughout the drawings, and a redundant description of the same elements is omitted.

Hereinafter, preferred embodiments of the present invention will be described in more detail with reference to the accompanying drawings.

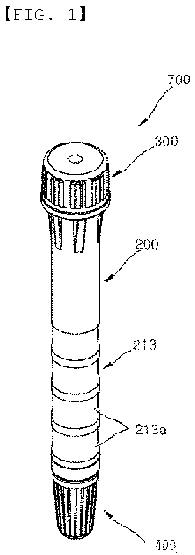

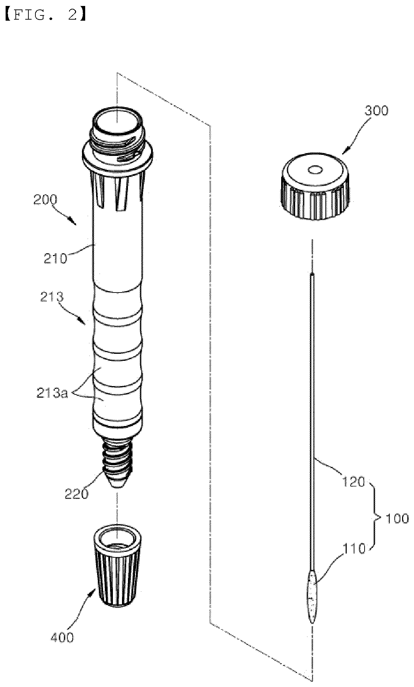

Referring to FIGs. 1 and 2, a specimen collection kit 700 according to an embodiment of the present invention may include a collection unit 100, a tube body 200, a cover member 300, a coupling member 400, and a specimen guiding portion 500.

First, the collection unit 100 is a unit that is used for collection of a specimen from an examinee by an examiner, and may include a head portion 110 configured to collect a specimen and a stick portion 120 coupled to the head portion 110 and configured to be gripped by an examiner. The collection unit 100 is formed in the shape of a cotton swab, but may be formed in various shapes and of various materials, so long as the same is capable of collecting a specimen. A detailed description thereof will be omitted.

The tube body 200 may be configured to accommodate the collection unit 100 containing the collected specimen in order to preserve and transport the collected specimen. An accommodation space 211, in which the collection unit 100 containing the collected specimen is accommodated, may be defined in the tube body 200, an outlet 221, which communicates with the accommodation space 211 and through which the specimen is discharged, may be formed in one end of the tube body 200, and an insertion hole 212, through which the collection unit 100 is inserted into the accommodation space 211, may be formed in the other end of the tube body 200.

In detail, the tube body 200 may include a body portion 210 and a coupling portion 220.

First, the insertion hole 212 may be formed in the other end of the body portion 210 so that the collection unit 100 is inserted into the body portion 210 therethrough, and the accommodation space 211 may be defined in the body portion 210 so that the body portion 210 accommodates the collection unit 100 inserted into the accommodation space 211.

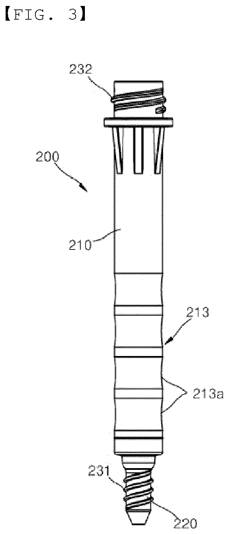

In this case, the cover member 300 may be detachably coupled to the other end of the body portion 210, and a threaded portion (refer to FIG. 3) for coupling with the cover member 300 may be formed on the outer circumferential surface of the other end of the body portion 210.

As illustrated, the body portion 210 may be formed in a cylindrical tube shape, but the embodiments are not limited thereto.

The body portion 210 may be formed of a transparent or translucent material so that an examiner is capable of visually checking the collection unit 100 in the body portion 210, and various materials such as a well-known resin-based material may be used for the body portion 210.

The coupling portion 220 may extend from one end of the body portion 210 in a longitudinal direction, and may be formed such that the coupling member 400 is coupled thereto.

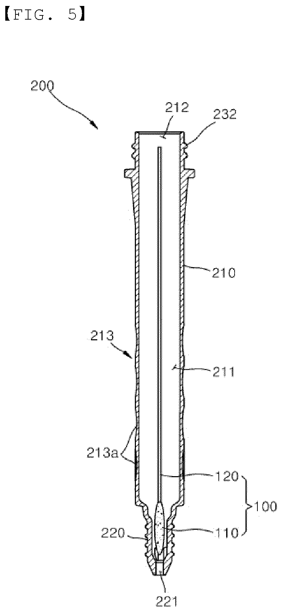

Here, the coupling portion 220 may include a distal end portion exposed to the outside, in which the outlet 221 is formed, and an inner passage 222 formed therein, through which the outlet 221 and the accommodation space 211 communicate with each other and through which the specimen is extracted (refer to FIG. 5). In this case, the inner passage 222 may be formed to have a cross-sectional flow area smaller than the cross-sectional flow area of the accommodation space 211 and larger than the cross-sectional flow area of the outlet 221.

Meanwhile, in the present invention, the tube body 200 may be formed of an elastically deformable soft material so that the collection unit 100 in the accommodation space 211 is pressed by external force.

The reason for this is to enable an examiner to press and squeeze the head portion 110 of the collection unit 100 with a reduced pressing force in the state in which the collection unit 100 is accommodated in the accommodation space 211 in the tube body 200.

Furthermore, the tube body 200 may include a pressing portion 213 so that the examiner more easily presses the collection unit 100 when applying external force to the tube body 200 from the outside.

The pressing portion 213 may be formed to have a thickness smaller than the thickness of another portion of the tube body 200 so that the examiner more easily performs a pressing task.

In addition, the pressing portion 213 may include a plurality of gripping grooves 213a formed in the outer side surface of the tube body 200. The gripping grooves 213a may serve to prevent slippage and improve a grip feeling, whereby the examiner may more easily press the tube body 200 and the tube body 200 may be readily pressed.

Meanwhile, the gripping grooves 213a may be formed in various shapes.

In this regard, referring to FIG. 3, the gripping grooves 213a may be formed in an annular shape in the circumferential direction of the tube body 200, and may be arranged so as to be spaced apart from each other in the longitudinal direction of the tube body 200. Here, the width and depth of each of the gripping grooves 213a may vary depending on the length and thickness of the tube body 200 and the set gripping position of the examiner.

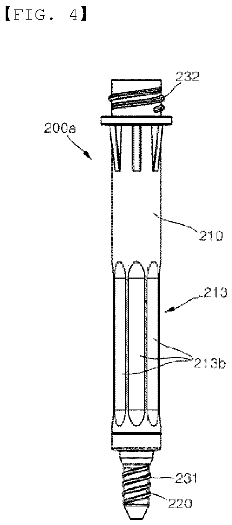

As an alternative to the embodiment shown in FIG. 3, referring to FIG. 4, the gripping grooves 213a may be formed in the longitudinal direction of a tube body 200a, and may be arranged so as to be spaced apart from each other in the circumferential direction of the tube body 200a. Similarly, the length and depth of each of the gripping grooves 213a may vary depending on the length and thickness of the tube body 200a. However, the embodiments are not limited thereto.

Meanwhile, although not illustrated, the inner side surface of a portion of the tube body 200, on which the pressing portion 213 is formed, may be bent toward the inner center of the tube body 200. The reason for this is to reduce a spacing distance between the inner side surface of the tube body 200 and the outer side surface of the head portion 110 in the accommodation space 211 and thus to reduce a pressing distance of the examiner, thereby further facilitating the pressing task. In this case, the bent portion of the tube body 200 may be formed in various shapes, so long as the same is capable of accomplishing the above purpose.

Furthermore, the tube body 200 may further include a pressing protrusion (not shown) protruding inwards from the inner side surface of the tube body 200 that is brought into contact with the head portion 110 when the pressing portion 213 is pressed. The pressing protrusion may serve to increase the pressing force applied to the head portion 110 by the examiner pressing the tube body 200, thereby further improving extraction of the specimen contained in the head portion 110. Here, the pressing protrusion may be formed in various shapes such as, for example, a well-known hemispherical shape.

The cover member 300 may be detachably coupled to the other end of the tube body 200 to open and close the insertion hole 212.

In the drawings, the cover member 300 is illustrated by way of example as being formed in a cup shape and detachably screwed to the tube body 200. In this case, the cover member 300 may have a threaded portion formed on the inner circumferential surface thereof so as to be engaged with the threaded portion 232 of the tube body 200.

The coupling member 400 may be detachably coupled to one end of the tube body 200 to open and close the outlet 221.

Referring to the drawings, the coupling member 400 may be detachably screwed to the coupling portion 220. The coupling member 400 may be formed in a cup shape, and may have a threaded portion formed on the inner circumferential surface thereof so as to be engaged with a threaded portion 220 formed on the outer circumferential surface of the coupling portion 220.

Here, as illustrated, each of the cover member 300 and the coupling member 400 may be screwed to the tube body 200 as a preferred embodiment for securing coupling force and preventing liquid leakage, but may be detachably coupled to the tube body 200 in various coupling manners such as a snap-fit manner.

Meanwhile, the specimen collection kit 700 described above is configured such that, when the examiner collects a specimen from an examinee using the collection unit 100 and inserts the collection unit 100 into the tube body 200, the tube body 200 stores the specimen in order to transport the same. In this case, since a reaction reagent is contained in the tube body 200, the specimen contained in the collection unit 100 and the reaction reagent are mixed with each other, and the mixture is extracted through the outlet 221 by the examiner.

However, the conventional specimen collection kit has a problem in that, when the examiner presses the tube body 200 to extract a specimen in the state in which the collection unit 100 is inserted into the tube body 200, the head portion 110 of the collection unit 100 blocks the outlet 221 and thus the specimen is not smoothly extracted through the outlet 221.

In other words, the conventional specimen collection kit has a problem in that it is difficult to extract a specimen due to clogging of the outlet 221 because the head portion 110 of the collection unit 100 blocks the outlet 221 during specimen extraction.

Therefore, the present invention includes the specimen guiding portion 500, which is provided in the tube body 200 in order to prevent blockage of the outlet 221 caused by the collection unit 100 during specimen extraction, thus ensuring smooth specimen extraction. Hereinafter, the configuration of the specimen guiding portion 500 and a principle of preventing blockage of the outlet 221 will be described.

First, the configuration of the specimen guiding portion 500 will be described. As described above, the specimen guiding portion 500 is provided on the inner side surface of the tube body 200 so as to be adjacent to the outlet 221, and serves to prevent blockage (clogging) of the outlet 221 caused by collection unit 100 inserted into the tube body 200, thereby inducing smooth flow of the specimen to the outlet 221.

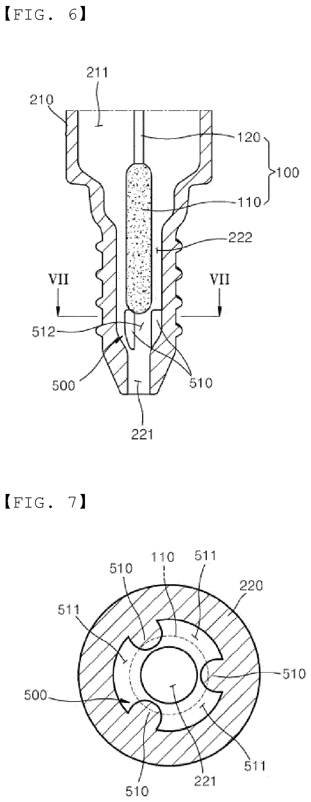

Referring to FIGs. 5 and 6, the specimen guiding portion 500 may include a plurality of protrusions 510 located in the inner passage 222 of the coupling portion 220 and formed so as to protrude from the inner circumferential surface of the coupling portion 220. The plurality of protrusions 510 may be disposed so as to be spaced apart from each other along the inner circumferential surface of the coupling portion 220 to form inter-protrusion passages 511 (refer to FIG. 7) therebetween, through which the specimen flows.

In this case, it is preferable that the protruding height of each of the protrusions 510 be not too high in order to prevent the occurrence of flow resistance of the specimen to the outlet 221. As shown in FIG. 7, the innermost portion of the inner side surface of each of the protrusions 510 may be located at a position farther outward than or aligned with the outer circumferential surface of the outlet 221.

Meanwhile, as illustrated, the cross-section of the inner side surface of each of the protrusions 510 may be formed in a round shape as a preferred embodiment, but may be formed in various other shapes, such as a flat shape.

Referring to FIG. 7, three protrusions 510 may be radially disposed so as to be spaced apart from each other at intervals of 120 degrees about a central axis about which the inter-protrusion passages 511 are formed, i.e. the central axis of the tube body 200. However, this is merely one example, and the number of protrusions 510 and the intervals therebetween may be diversely changed.

Hereinafter, the principle of preventing blockage of the outlet 221 using the specimen guiding portion 500 and detailed configuration conditions of the tube body 200 and the specimen guiding portion 500 for realizing the principle will be described.

First, in order to be easily inserted into the accommodation space 211 in the tube body 200, the head portion 110 may be formed to have a cross-sectional area smaller than the cross-sectional area of the accommodation space 211 in a direction perpendicular to the longitudinal direction of the tube body 200.

In addition, the head portion 110 may be formed to have a cross-sectional area smaller than the cross-sectional area of the inner passage 222 of the coupling portion 220 so that the head portion 110 is received in the inner passage 222 and comes into contact with the reaction reagent contained in the inner passage 222.

In addition, the head portion 110 may be formed to have a cross-sectional area larger than the cross-sectional area of the outlet 221 so as not to be inserted into the outlet 221.

Furthermore, it is preferable for the head portion 110 to be formed to have a larger cross-sectional area than a center passage 512, which is defined by the inner side surfaces of the protrusions 510, so as not to be inserted into a region surrounded by the protrusions 510. If the head portion 110 is inserted into the center passage 512 surrounded by the protrusions 510 and then is placed at the distal end portion in which the outlet 221 is formed, the head portion 110 may block not only the outlet 221 but also the inter-protrusion passages 511 between the protrusions 510, thereby making it difficult to extract the specimen.

Meanwhile, the center passage 512, which is a space defined by the inner side surfaces of the protrusions 510, is a cylindrical center channel having the same central axis as the outlet 221. Although the center passage 512 is illustrated in the drawings as being formed to have substantially the same size as the outlet 221, the size and shape of the center passage 512 may be diversely changed, so long as the above-described conditions are satisfied.

Therefore, according to the above-described conditions, in the specimen collection kit 700 of the present invention, the collection unit 100 is caught by the inner end portions of the protrusions 510 of the specimen guiding portion 500, whereby it is possible to prevent blockage of the outlet 221 due to insertion of the head portion 110 into the outlet 221. In addition, even when the head portion 110 comes into contact with the specimen guiding portion 500, the specimen flows to the outlet 221 through the inter-protrusion passages 511 between the protrusions 510 and accordingly, is smoothly extracted.

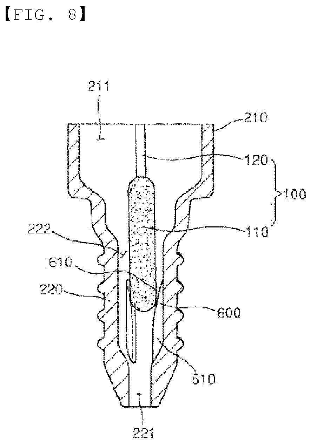

FIG. 8 is a view showing another embodiment of the present invention. The specimen collection kit 700 according to this embodiment of the present invention may further include a fitting portion 600 configured not only to prevent blockage of the outlet 221 due to the collection unit 100, but also to fix the position of the collection unit 100 in the tube body 200.

The fitting portion 600 may extend from the side of the specimen guiding portion 500 that is opposite the outlet 221 (the upper side in the drawing) toward the accommodation space 211 in the longitudinal direction of the tube body 200 so that the head portion 110 of the collection unit 100 is fitted thereto and supported thereby.

Here, the fitting portion 600 may integrally extend from each of the protrusions 510, and may extend in the longitudinal direction of the protrusions 510 so as not to block the inter-protrusion passages 511.

Furthermore, the fitting portion 600 may be formed to have an inclined surface 610 that is inclined downward from the inner circumferential surface of the coupling portion 220 toward the interior of the coupling portion 220, whereby the head portion 110 may be fitted to the fitting portion 600 regardless of the size of the head portion 110, and fitting of the head portion 110 may be further facilitated.

As described above, the specimen collection kit 700 according to the embodiment of the present invention allows the head portion 110 to be held at a preset central position without shaking in the tube body 200 during storage and transportation, thereby providing sufficient mixing between the specimen and the reaction reagent and more effective specimen extraction.

Although the present invention has been described with reference to embodiments illustrated in the drawings, it is to be understood that the foregoing embodiments are merely exemplary and various modifications or equivalent embodiments thereof may be made from the detailed description of the present invention by those skilled in the art. Accordingly, the true technical scope of the present invention should be defined by the technical spirit of the appended claims.

### [Industrial Applicability]

The present invention is applicable to specimen collection instruments.

## Claims

1. A specimen collection kit comprising:
a tube body comprising an accommodation space defined therein to accommodate a collection unit containing a collected specimen, an outlet formed in one end thereof so as to communicate with the accommodation space to discharge the specimen, and an insertion hole formed in another end thereof to allow the collection unit to be inserted into the accommodation space;
a cover member detachably coupled to the other end of the tube body to open and close the insertion hole;
a coupling member detachably coupled to the one end of the tube body to open and close the outlet; and
a specimen guiding portion provided on an inner side surface of the tube body so as to be adjacent to the outlet to prevent blockage of the outlet due to the collection unit and to induce flow of the specimen to the outlet.

2. The specimen collection kit according to claim 1, wherein the tube body is formed of an elastically deformable soft material to allow the collection unit in the accommodation space to be pressed by external force.

3. The specimen collection kit according to claim 2, wherein the tube body comprises a pressing portion configured to enable an examiner to press the collection unit by applying external force to the pressing portion from outside, and
wherein the pressing portion is formed to have a smaller thickness than the tube body.

4. The specimen collection kit according to claim 2, wherein the tube body comprises a pressing portion configured to enable an examiner to press the collection unit by applying external force to the pressing portion from outside, and
wherein the pressing portion comprises a plurality of gripping grooves formed in an outer side surface of the tube body.

5. The specimen collection kit according to claim 4, wherein the gripping grooves are formed in a longitudinal direction of the tube body and are arranged so as to be spaced apart from each other in a circumferential direction of the tube body.

6. The specimen collection kit according to claim 4, wherein the gripping grooves are formed in a circumferential direction of the tube body and are arranged so as to be spaced apart from each other in a longitudinal direction of the tube body.

7. The specimen collection kit according to claim 1, wherein the tube body comprises:
a body portion comprising the accommodation space defined therein and the insertion hole formed in another end thereof, the body portion being configured to allow the cover member to be coupled thereto; and
a coupling portion extending from one end of the body portion in a longitudinal direction, the coupling portion comprising the outlet formed in an exposed distal end portion thereof and an inner passage formed therein to allow the outlet and the accommodation space to communicate with each other, the inner passage having a cross-sectional flow area smaller than a cross-sectional flow area of the accommodation space and larger than a cross-sectional flow area of the outlet, the coupling portion being configured to allow the coupling member to be coupled thereto.

8. The specimen collection kit according to claim 7, wherein the specimen guiding portion comprises a plurality of protrusions located in the inner passage, formed so as to protrude from an inner circumferential surface of the coupling portion, and disposed so as to be spaced apart from each other along the inner circumferential surface of the coupling portion to form inter-protrusion passages therebetween to allow the specimen to flow through the inter-protrusion passages.
